# EUROPEAN PATENT APPLICATION

(11) **EP 1 208 823 A1**
(43) Date of publication of application: **29.05.2002**
(21) Application number: 00125847.4
(22) Date of filing: 25.11.2000
(51) Int. Cl.: A61F 13/15

(54) **Absorbent articles with asymmetric flaps**

(71) Applicant: THE PROCTER & GAMBLE COMPANY, Cincinnati, Ohio 45202 (US)
(72) Inventor: Hohmann, Sonja, 65812 Bad Soden (DE); Kitzinger, Ulrike, 65529 Waldems (DE); Soldato, Daniela, 66010 Casacanditella, (Chieti) (IT)
(74) Representative: Hirsch, Uwe Thomas M.H.

(57) **Abstract**

The present invention relates to disposable absorbent articles, such as female sanitary napkins, panty liners and adult incontinence devices. The present invention concerns such disposable absorbent articles having flaps such that at least one flap overlaps the central longitudinal axis of the absorbent article when it is in an in-use folded configuration as defined herein. According to the present invention either the arrangement or shape of the flaps or both the arrangement and shape of the flaps is such that the absorbent article when it is in an unfolded configuration is not symmetric about its central longitudinal axis. In the in-use folded configuration the flaps are such that they do not overlap. The benefit of the invention is that the width of the flaps allows strong and effective adhesion to the undergarment and soiling protection of the inner rim of the undergarment. These benefits are obtained while the flaps are not overlapping which in particular facilitates easy and destructive free removal of the used and soiled absorbent article.

## Description

### Field of the Invention

The present invention relates to disposable absorbent articles, such as female sanitary napkins, panty liners and adult incontinence devices. The present invention concerns such disposable absorbent articles having flaps such that at least one flap overlaps the central longitudinal axis of the absorbent article when it is in an in-use folded configuration as defined herein. According to the present invention either the arrangement or shape of the flaps or both the arrangement and shape of the flaps is such that the absorbent article when it is in a unfolded configuration is not symmetric about its central longitudinal axis. In the in-use folded configuration the flaps are such that they do not overlap. The benefit of the invention is that the width of the flaps allows strong and effective adhesion to the undergarment and soiling protection of the inner rim of the undergarment. These benefits are obtained while the flaps are not overlapping which in particular facilitates easy and destructive free removal of the used and soiled absorbent article.

### Background of the Invention

Absorbent articles, particularly sanitary napkins or panty liners, having flaps are disclosed in the literature and are available in the market place. The flaps help stabilize the absorbent article from shifting out of place and provide additional protection for the edges of the wearer's undergarments about which they are folded. Increasing the width of the flaps results in a larger area becoming available for adhesion between the flaps and the undergarment further stabilizing the absorbent article. In current commercial embodiments the flaps are identical in shape and arranged such that the absorbent article is symmetrical about its central longitudinal axis when in the non-folded state. If the flaps have a width such that they overlap the central longitudinal axis of the absorbent article when in their in-use folded configuration, then the flaps overlap in this configuration. Adhesion of the flaps to each other, despite being well known in the written art on sanitary napkins, prevents easy removal of the absorbent article. This can even result in the soiled product being torn on removal resulting in an unpleasant experience for the wearer. This problem is particularly severe for narrow absorbent articles for use in undergarments comprising a narrow crotch piece. For such undergarments the area available for flap adhesion is small, necessitating that the flaps overlap the central longitudinal axis of the absorbent article when in the in use folded configuration in order to adhere well in this configuration. This results in a trade off between ease of removal and sufficient adhesion of the absorbent article, particularly the flaps, to the undergarment.

Absorbent articles having flaps of various types are disclosed in U.S. Patent 4,589,876, entitled "Sanitary Napkin", which issued to Van Tilburg on May 20, 1986, U.S. Patent 4,285,343, entitled "Sanitary Napkin", which issued to McNair on Aug 25, 1981, U.S. Patent 4,900,320, entitled "Sanitary Napkin with Panty Gathering Flaps", which issued to McCoy on Feb 13, 1990, U.S. Patent 5,125,918, entitles "Sanitary Napkin having an attachment system comprising biased hinges", which issued to Seidy on Jun 30 1992, U.S. Patent 4, 608, 047, entitled "Sanitary Napkin attachment means", which issued to Mattingly on Aug 26 1986, US Patent 3, 397, 697, entitled "Disposable Sanitary Shield for Undergarments", issued to Rickard on Aug 20, 1968, European Patent 0 467 184 A1, entitled "Form retentive absorbent pads", to Davis et al, published on Jan 22, 1992, European Patent 0675 704 A1, to Leeker, published on Oct 11 1995. Further other publications such as UK design 2 076 491, German design model 499 00 112.5, German design model 499001001, International design DM/045 544, UK design 1 049 870 and German Utility model 299 21 095.2 of March 30, 2000 describe and illustrate as preferred embodiments absorbent articles which are symmetrical in their unfolded states. In addition all said publications do not realize and are not concerned with the problem underlying the present invention off effective adhesion and ease of removal by prevention of flap overlap.

The solution offered by US patent 5 713 886 is in the context of a panty liner for a thong type undergarment, which as a fundamental aspect has a very narrow width in its rear portion. In this disclosure the panty liner has a total of 3 flaps with a single flap along one side of the narrow rear portion of the panty liner thus leaving the other side unattached and unprotected. This suggested solution is not satisfactory because the single sided attachment can cause unsatisfactory attachment or delamination during use, which in turn causes shifts of the article in the undergarment. Further the design is not satisfactory because the panty liner does not provide any protection against soiling for the undergarment on at least one side of the rear portion.

Accordingly there is a need for an improved design for absorbent articles having flaps in which at least one flap overlaps the central longitudinal axis of the absorbent article when in the in use folded configuration without attaching the flaps to each other.

### Summary of the Invention

The present invention relates to an absorbent article having an in use folded configuration and a non-folded configuration. The absorbent article comprises an central absorbent pad having a body facing side, a garment facing side, longitudinally extending edges, transverse ends, a central longitudinal axis and a transverse axis. Each longitudinal edge of the central absorbent pad has a flap extending from it. The flaps are such that at least one flap overlaps the central longitudinal axis of the absorbent article when in the in use folded configuration. In addition either the arrangement or shape of said flaps or both the arrangement and shape of said flaps is such that the absorbent article when in the non-folded state is not symmetric about its central longitudinal axis. The flaps are such that they do not overlap when in the in use folded configuration.

The absorbent article of the present invention provides at least two benefits. The present invention allows effective adhesion to the users undergarment as the width of at least one of the flaps is such that it overlaps the central longitudinal axis of the absorbent article when in the in use folded configuration. This benefit is particularly important for narrow absorbent articles for use in undergarment consisting of a narrow crotch piece. In this situation the area available for flap adhesion is small and wide flaps are required to fix the absorbent article effectively in place. The arrangement or shape of said flaps or both the arrangement and shape of said flaps in the present invention is such that the absorbent article when in its non folded state is not symmetric about its central longitudinal axis, and hence said flaps are able to adhere to the underside of the users undergarment without overlap. This provides the second benefit of ease of removal, as the flaps have not adhered to each other.

### Brief Description of the Drawings

Fig 1. Is a plan view of a preferred absorbent article of the present invention with offset flaps.
Fig 2. Is a plan view of a preferred absorbent article of the present invention with offset and non-identical flaps.
Fig 3. Is a sectional view taken across the line of the transverse axis 2 in Fig 1.

### Description of the Preferred Embodiments

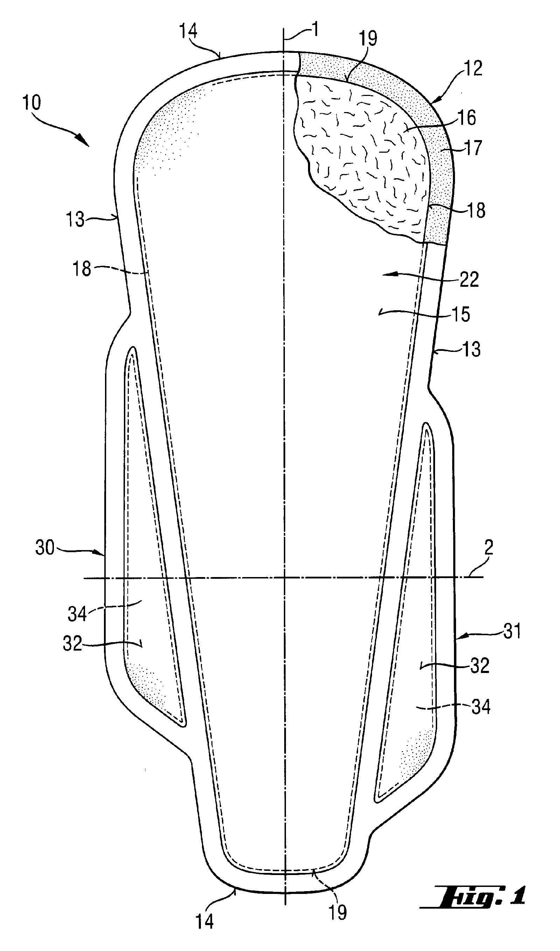

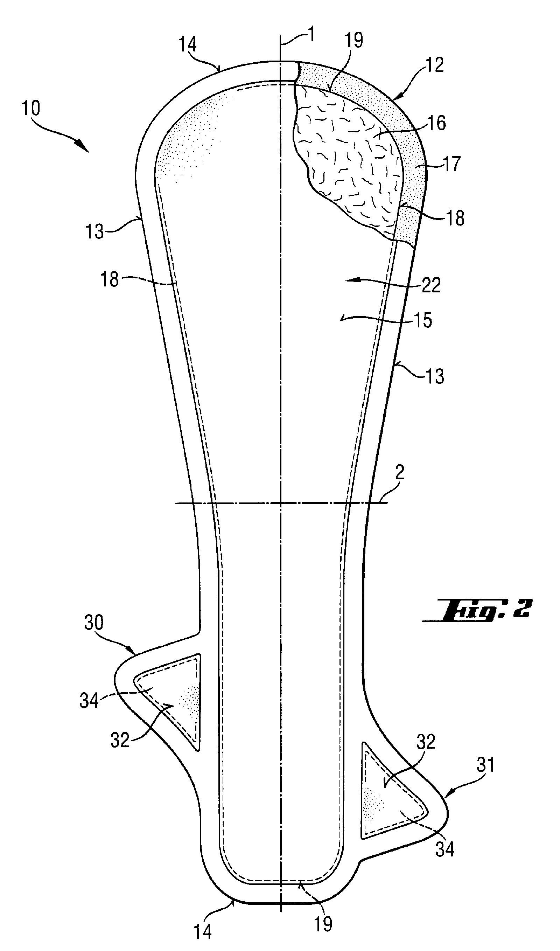

As used herein, the term "longitudinal" refers to the direction of the central longitudinal axis referred to by reference numeral 1 in Fig 1 and 2. As used herein the term "transverse" refers to the direction of the transverse axis referred to by reference numeral 2 in Fig 1 and 2 and which is generally perpendicular to the central longitudinal axis.

The wording in use folded configuration conventionally refers to the in use configuration in which the absorbent article is firmly placed in the inner crotch portion of an undergarment, such that the flaps are folded about the crotch portion through the leg openings so as to overlie the outer crotch portion of the undergarment and are secured in such a position. However this presupposes a particular match of dimensions between article and undergarment and hence for clarity in the context of the present application the term "in use folded configuration", where used to define the invention, will refer to the configuration of an article in which the flaps are folded along a line delimiting the absorbent core and the flaps onto the garment facing side of the article. In case the delimiting line is not linear the closest possible fold along the delimiting line should be used instead. As used herein, the term "non folded configuration" or "unfolded configuration" refers to the configuration in which the flaps of the absorbent article are in the same plane as the absorbent article as illustrated in Fig 1 and 2.

The preferred embodiments of the absorbent article of the present invention are shown in Fig 1 and Fig 2. The absorbent articles in Fig 1 and Fig 2 are generally referred to by reference numeral 10. Absorbent article 10 comprises a central absorbent pad that is generally referred to by reference numeral 12. The central absorbent pad 12 has longitudinal edges 13 generally oriented parallel to the central longitudinal axis 1, transverse ends 14 generally oriented parallel to the transverse axis 2, a body facing side 22 and an undergarment facing side 24. Extending from each longitudinal edge 13 of the central absorbent pad 12 are flaps 30 and 31.

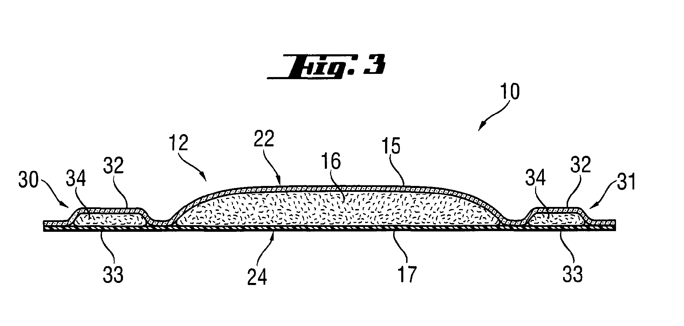

The central absorbent pad 12 usually will comprise a liquid pervious top sheet 15, an absorbent core 16, and a liquid impervious back sheet 17 as shown in Fig 3. However other configurations and designs in which the top sheet or the back sheet or both are integral parts of the absorbent core, are simply not provided or wherein the absorbent core is an integral part of another element of the article are also considered useful in the context of the present invention. The central absorbent pad 12 can be essentially of any dimension. However, in a preferred embodiment the absorbent element comprises a wider front rear portion and a narrower central portion so that it may be used in undergarments with narrow crotch regions. In an alternative preferred embodiment a generally thong or triangular shaped article having a wider front and narrow rear portion is considered especially for use in modern undergarments of the G-string or string tanga type.

The liquid pervious top sheet 15, if present should be compliant, soft feeling and non-irritating to the user's skin. It can be made from any of the conventional materials for this type of use. Examples of suitable materials are woven and nonwoven polyester, polypropylene, nylon and apertured films, preferably 3 dimensionally apertured films. The outer surface of the top sheet, preferably around the locations of liquid penetration may be treated with a compound that renders the surface more hydrophilic allowing the liquid to penetrate the top sheet 15 easily. Alternatively the outer surface of the top sheet, preferably between the locations of liquid penetration, may be treated with a compound which renders the surface more hydrophobic allowing liquid deposited there to migrate fast to locations where it can penetrate the top sheet 15 easily, thus reducing liquid build up on the top sheet. The inner surface of the top sheet may be secured to the surface of the absorbent core 16 for example by the use of an adhesive or by other bonding means such as compression and/or thermo-bonding. This contacting relationship also results in liquid penetrating the top sheet 15 easier.

The absorbent core 16 if present is positioned between the top sheet 15 and the back sheet 17 and comprises a fluid absorbing material. Fluid absorbing materials may be natural or synthetic or combinations thereof. Examples of absorbent materials include cellulose fibers and super-absorbent gelling materials. Absorbent materials may be used either alone or in combination with other materials, both absorbent or not. The absorbent core can be provided as a single entity or comprise several layers. The absorbent core 16 has longitudinal edges 18 generally oriented parallel to the central longitudinal axis 1, and transverse ends 19 generally oriented parallel to the transverse axis 2

The back sheet 17 if present is impervious to liquids or retarding liquid penetration sufficient for the intended purpose of preventing fluid from the absorbent core migrating towards an underlying garment and soiling the garment or body of the user. The back sheet can be made from any of the conventional materials for this type of use. Suitable materials include embossed or non-embossed polyethylene films and especially breathable materials or multiple layer composites.

Preferably a portion of the undergarment facing side of the back sheet 24 is coated with a panty-fastening adhesive. The adhesive is intended to fix the central absorbent pad to the crotch of the undergarment during use of the article. Any adhesive used in the art for such purpose can be used herein, with pressure sensitive adhesives being preferred. Before the absorbent article 10 is placed in use the adhesive should be covered by a removable release liner to prevent the adhesive from drying out, being contaminated, or sticking to a surface other than intended.

The articles according to the present invention further comprise flaps (30,31) which are folded about the edge of the undergarment's leg openings in order to provide additional protection from soiling the undergarment and, provided the flaps are equipped with an attachment means such as the panty fastening adhesive mentioned above or a mechanical attachment means, to support fixation of the article to the undergarment during use. The flaps (30,31) can be made of any suitable material. They can be integral extensions of one, some or all of the materials used to provide the central absorbent pad (12). Alternatively the flaps (30, 31) can be constructed separately from the absorbent central pad (12). In this case they can be made from the same materials used already for the absorbent pad or form different materials such as those mentioned herein already. If provided separately the flaps are attached to the central absorbent pad (12) by any means conventionally used in combining absorbent articles, such as adhesives, thermo or mechanical bonding. A key benefit of the integral construction is the ease of manufacture while the attached flaps provide benefits in material consumption and flexibility in material selection for the article manufacturer.

In an embodiment according to the present invention the flaps (30 and 31) comprise a top sheet (32), a back sheet 33 and optionally also comprise an absorbent core 34. In a preferred embodiment the flap top sheet (32) is also integral with the material that is used for top sheet (15). In another preferred embodiment the flap back sheet (33) is also integral with the material that is used for back sheet (17). In a preferred embodiment for use as a thong shaped sanitary napkin no absorbent material or only a very thin absorbent material such as a tissue is provided as absorbent material in the flaps.

Flaps (30 and 31), can be essentially of any shape provided that at least one flap overlaps the central longitudinal axis of the absorbent article when in the in use folded configuration. As illustrated in Fig 1 the flaps (30 and 31) may have the same shape but be located at different locations along the longitudinal edges (13) such that they do not overlap when in the in use folded configuration. As illustrated in Fig 2 the flaps may have a different shape to achieve this purpose. In Fig 2 the different shape of flap (30) is achieved by using a transverse mirror image of flap (31). Any differing shapes between the flaps may be used provided they do not overlap in the in use folded configuration. Flaps (30 and 31) may be different shapes and also be located at different distances along the longitudinal edges (13) as illustrated in Fig. 2. The flaps (30, 31) can extend along a small or a long portion of the central absorbent pad (12). It is preferred that the flaps be as long as practical so as to maximize the extent of undergarment side edge that is covered. In this respect it is possible and preferred that one or even both flaps (30, 31) extend all the way to the rear end of the absorbent article.

As indicated above the most beneficial use of the present invention is in the context of articles such as sanitary napkins or panty liners intended to be used in undergarments having a narrow portion such as undergarments with narrow crotch width or G-string undergarments and which articles hence also have a narrow crotch width or a thong shaped design. Especially string shaped undergarments and with them thong shaped sanitary napkins and panty liners have recently become fashionable, especially in non-white coloring. It is therefore within the scope of the present invention to provide such napkins or panty liners with features suitable for the aesthetic, cosmetic and discretion aspects triggering the use of string undergarments such as general fashion colors besides white, especially black, for all or some part of the article. In particular providing articles at least with flaps that are colored so as to maintain the visual discretion achieved by the use of a thong article in a string undergarment by providing them in a color matching the color of the undergarment is an especially appreciated article configuration. In this context the use of a visually impenetrable dark color, such as black has been found most preferred for discreteness, ease of manufacturing (besides conventional white coloring black is the strongest visually detectable color for visual inspection and control equipment, especially for such sensors which do not use color), proven safety of the coloring components (carbon) and acceptance by the user (black undergarments are the most frequently used colored undergarments besides white).

Naturally any modification conventional in the art to the components or materials or construction of the absorbent articles according to the present invention, provided the inventive aspect of the asymmetric flap design as included in the appended claims are preserved, will be at the discretion of the skilled practitioner and will not deviate from the present invention.

## Claims

1. An absorbent article (10) comprising an central absorbent pad (12) having a body facing side, a garment facing side, a central longitudinal axis (1) and a transverse axis (2), longitudinal edges (13) extending in a generally longitudinal direction, and transverse ends (14) extending in a generally transverse direction, and said absorbent article (10) further comprising flaps (30, 31) extending from each of said longitudinal edges (13) of said central absorbent pad (12), and said flaps (30,31) having an in use folded configuration and a non folded configuration, and said flaps (30,31) being **characterized**
**in that** at least one flap (30, 31) overlaps the central longitudinal axis of the absorbent article when in the in use folded configuration, and in that the arrangement or shape or both the arrangement and the shape of said flaps (30,31) is such that the absorbent article (10) when viewed in the non folded configuration is not symmetric about said central longitudinal axis (1),
and **in that** the two flaps (30,31) do not overlap when they are in the in use folded configuration.

2. An absorbent article according to claim 1 **characterized in that** said article has a thong shape comprising a central absorbent pad (12) comprising a front portion and a rear portion, said front portion is wider in transverse extension than said rear portion.

3. An absorbent article according to any of the preceding claims **characterized in that** said flaps (30 and 31) are located at different locations along the longitudinal edges (13).

4. An absorbent article according to any of the preceding claims **characterized in that** said flaps (30 and 31) are provided with a different shape wherein the shape of one flap (30) is achieved by using a transverse mirror image of the other flap (31).

5. An absorbent article according to any of the preceding claims **characterized in that** said article (10) comprises a top sheet (15) and a back sheet (17) and **in that** said flaps (30 and 31) are provided as integral extensions of said top sheet (15) and said back sheet (17).

6. An absorbent article according to any of the preceding claims **characterized in that** said flaps (30 and 31) are provided with a flap attachment means, preferably an adhesive flap attachment means.

7. An absorbent article according to any of the preceding claims **characterized in that** at least said flaps (30 and 31) are provided in a color suitable to camouflage said flaps (30 and 31) when said absorbent article is in use, preferably said color is black or red.

8. An absorbent article according to any of the proceeding claims **characterized in that** said article is a sanitary napkin or a panty liner.
